# EUROPÄISCHE PATENTSCHRIFT

(11) **EP 0 632 827 B1**
(45) Veröffentlichungstag und Bekanntmachung des Hinweises auf die Patenterteilung: **16.04.1997**
(21) Anmeldenummer: 93905304.7
(22) Anmeldetag: 05.03.1993
(51) Int. Cl.: C12M 3/06, C12M 1/12, C12N 1/38, C12N 1/00

(54) **VERFAHREN ZUR ERZEUGUNG HOHER ZELLDICHTEN**
METHOD FOR PRODUCING HIGH CELL DENSITIES
PROCEDE DE PRODUCTION DE CONCENTRATIONS CELLULAIRES ELEVEES

(30) Priorität: 17.03.1992 DE 4208429
(43) Veröffentlichungstag der Anmeldung: 11.01.1995
(73) Patentinhaber: Märkl, Herbert, Prof. Dr.-Ing., D-21218 Seevetal (DE)
(72) Erfinder: Märkl, Herbert, Prof. Dr.-Ing., D-21218 Seevetal (DE)
(74) Vertreter: Schmidt-Bogatzky, Jürgen, Dr. Ing.
(86) Internationale Anmeldenummer: EP9300503
(87) Internationale Veröffentlichungsnummer: WO9319161

(56) Entgegenhaltungen:
- EP-A- 0 242 984
- EP-A- 0 419 234
- US-A- 3 418 208
- US-A- 3 816 261
- CHEMICAL ABSTRACTS, Band 75, Nr. 7, 16. August 1971, Columbus, OH (US); H. TONE et al., Seite 238, Nr. 47505g/
- PATENT ABSTRACTS OF JAPAN, Band 12, Nr. 365 (C-532)(3212), 29. September 1988/
- Journal of Fermentation and Bioengineering, Vol.69,Nr.4,S.244-49;1990

## Beschreibung

Die Erfindung betrifft ein Verfahren zur Erzeugung hoher Zelldichten in einem Reaktionssystem, das aus mindestens zwei Kammern besteht, die über mindestens eine Membran miteinander gekoppelt sind und bei dem die mit Nährstoffen zu versorgende Kultur von Mikroorganismen oder pflanzlichen oder tierischen Zellen sich in der einen Kammer befindet und die wachstumshemmenden Stoffwechselprodukte der Kammer aus dieser Kammer durch die Membran in die Lösung der anderen Kammer gelangen, und eine Vorrichtung zur Durchführung des Verfahrens.

Wenn z. B. bestimmte Produkte mit Mikroorganismen erzeugt werden sollen, wird oft eine hohe Organismendichte angestrebt, da die Konzentration des erzeugten Produktes in der Regel mit der Menge der produzierten Mikroorganismen korreliert. Um hohe Organismenkonzentrationen zu erreichen, ist es bekannt, die Organismen zunächst in einem üblichen "batch-Verfahren" anzuziehen und dann, wenn die auch als Substrat bezeichneten eingesetzten Nährstoffe aufgebraucht sind, mit konzentrierten Nährstoffen nachzufüttern, was auch als "fed-batch-Verfahren" bezeichnet wird. Produziert man z. B. E. coli Bakterien auf der Basis eines Substrates, dessen Kohlenstoffquelle aus Glycerin besteht, so erzielt man auf die angegebene Weise Organismendichten, die bei etwa 60 g Trockengewicht pro Liter Kulturvolumen liegen. Höhere Konzentrationen werden auf diese Weise nicht erreicht. Ursächlich hierfür ist, daß beim Wachstum der Organismen gewisse Stoffe (Produkte) entstehen, die sich anreichern und weiteres Wachstum verhindern. Dieser Vorgang ist unter dem Begriff "Produkthemmung" bekannt.

Zur Erhöhung der Zelldichte ist es somit erforderlich, die Produkthemmung abzubauen. Dies kann beispielsweise durch den kontinuierlichen Abzug von Nährlösung über ein Membranmodul erreicht werden. Die eingesetzte Membran verhindert hierbei den Verlust von Mikroorganismen. Bei einer bekannten Ausführung befindet sich das Membranmodul außerhalb des Reaktors und wird im Kreislauf von der Reaktorflüssigkeit durchströmt. Diese Anordnung hat jedoch den technischen Nachteil, daß die eingesetzte Membran, obwohl sie im Kreuzstrom-Betrieb geschaltet ist, durch die zurückgehaltenen Mikroorganismen teilweise verstopft wird und damit der transmembrane Fluß mit der Zeit abnimmt. Nachteilig ist auch, daß sich ständig ein Teil der Organismen außerhalb des Kulturgefäßes befindet und dort weder ausreichend mit Sauerstoff noch mit Nährsubstanzen vorsorgt wird. Dies hat die Folge, daß in manchen Fällen die Produktion gewünschter Wertstoffe in dieser bekannten Anordnung sehr stark reduziert wird. Darüber hinaus ist bei manchen Organismenarten durch mechanische Einflüsse der Pumpe eine nachteilige Schädigung der Zellen zu erwarten.

Durch die ZS "Journal of Fermentation and Bioengineering", Vol. 69, Nr. 4, S. 244-249, 1990 ist eine weitere Anordnung bekannt, in der die Membran in den Reaktor integriert ist und hierdurch der Reaktor aus zwei gut durchmischten Kammern besteht. Die erste dient der Bereitstellung von frischem Nährmedium und die zweite der Kultur von Mikroorganismen. Die Kultur wird über die Membran durch Diffusionsmechanismen mit Nährstoffen versorgt, wobei gleichzeitig wachstumshemmende Stoffe über die Membran abgezogen werden. Durch einen ständigen Austausch von Nährmedium in der ersten Kammer wird eine gute Ver- und Entsorgung der Kultur erreicht. Werden die beteiligten Reaktorräume gut durchmischt, so besteht erfahrungsgemäß für die Membran keine Verstopfungsgefahr, da über die Membran kein hydraulischer Strom geführt wird. Stoffaustausch findet wesentlich über Diffusion statt. Ein weiterer Vorteil dieser Anordnung besteht darin, daß auch Membranen eingesetzt werden können, die keinen hydraulischen Fluß zulassen. Solche auch als Dialysemembranen bezeichnete Trennelemente halten auch etwaige extracelluläre Produkte zurück, sofern diese ein gewisses Molekulargewicht übersteigen. So kann beispielsweise eine Cellulosemembran extracelluläre Enzyme mit einem Molekulargewicht von größer als 10.000 zurückhalten, während wachstumshemmende Stoffe durchgelassen und damit aus dem System ausgeschleust werden. Der Nachteil dieser Anordnung besteht jedoch darin, daß die gewünschten günstigen Verhältnisse nur dadurch geschaffen werden können, wenn die in der ersten Kammer enthaltene Nährlösung ständig erneuert wird. Auf diese Weise wird zusätzliche Nährlösung verbraucht, die nicht direkt dem Wachstum von Organismen dient. Bezogen auf das erzeugte Produkt verschlechtert sich somit die Nährstoffbilanz.

Nach der EP-A-04 19 234 ist ferner ein Reaktor zur Züchtung von Zellkulturen bekannt, der hierzu ein Membranmodul aufweist. Durch das Membranmodul wird eine salzhaltige Dialyseflüssigkeit geführt, wobei wachstumshemmende Stoffwechselprodukte aus der Zellzuchtkammer aufgenommen und abtransportiert werden. Hierbei besteht der Nachteil, daß nur ein Teil der Dialyseflüssigkeit wieder verwendet wird, während der verbleibende Teil abgeführt wird und für den Betrieb des Reaktors verlorengeht, so daß die Dialyseflüssigkeit ersetzt werden muß. Der nach der EP-A-0 419 234 bekannte Reaktor weist ferner keine mit Rührern versehene gut durchmischte Kammern auf. Hierdurch besteht die Gefahr, daß sich nach einer gewissen Zeit auf den eingesetzten Membranen Deckschichten ausbilden, die die Transportleistung der Membranen vermindern. Im übrigen kann ohne Rührwerke keine für die Erzeugung von schnell wachsenden Mikroorganismen notwendige größere Menge an Sauerstoff eingetragen werden.

Die Aufgabe der Erfindung besteht darin, das Verfahren der eingangs genannten Art so zu verbessern, daß bei ökonomisch günstiger Nährstoffversorgung ein Wachstum auch extrem dichter Kulturen ermöglicht und der Einfluß niedermolekularer das Wachstum hemmender Stoffe stark vermindert wird, und ferner gleichzeitig eine Vorrichtung zur Durchführung des verbesserten Verfahrens aufzuzeigen. Sofern extracelluläre hochmolekulare Wertstoffe im Einzelfall entstehen, sollen diese ferner angereichert werden.

Erfindungsgemäß erfolgt die Lösung der Aufgabe bezüglich des Verfahrens durch die kennzeichnenden Merkmale des Anspruchs 1 und bezüglich der Vorrichtung durch die kennzeichnenden Merkmale des Anspruchs 16. Vorteilhafte Ausgestaltungen der Erfindung werden in den abhängigen Ansprüchen beschrieben. Nach der Erfindung ist die Erzeugung außerordentlich hoher Zelldichten in Kulturen von Mikroorganismen von Mikroorganismen oder pflanzlichen Zellen oder tierischen Zellen möglich.

Die Vorteile der Erfindung werden am Beispiel der folgenden Versuche näher erläutert.

### Versuch 1:

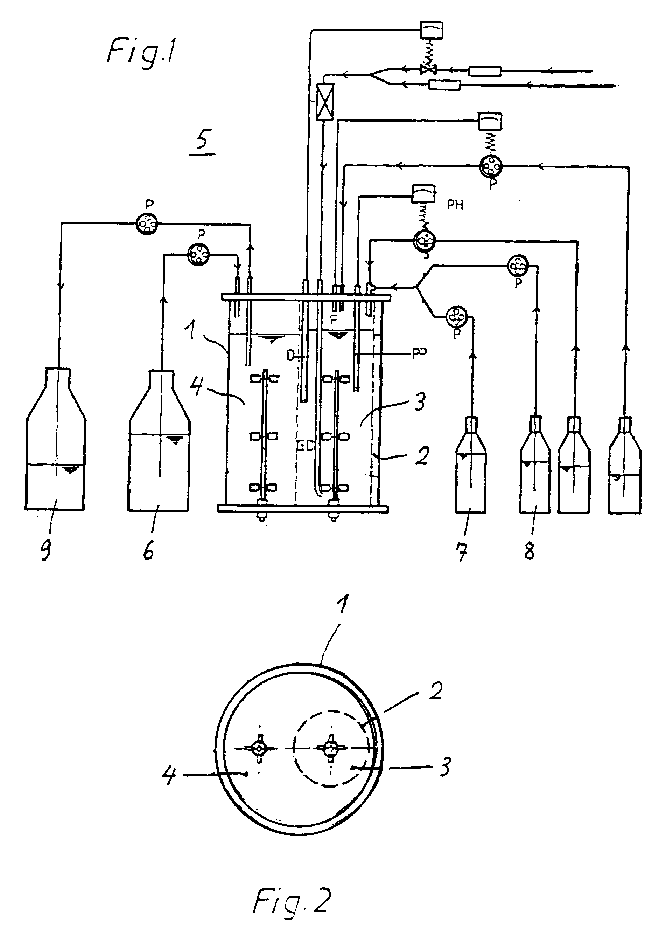

Es wurde ein Dialyse-Bioreaktor 1 in einer Versuchsanordnung 5 wie in Fig. 1 dargestellt verwendet. Figur 2 zeigt die Ansicht eines Schnitts, senkrecht zur Reaktorachse. Der Innenraum des Dialyse-Bioreaktors 1 ist durch eine zylinderförmige Membran 2 in zwei getrennte Reaktionsräume so aufgeteilt, daß eine innere Kammer 3 in dem Membranzylinder und eine äußere Kammer 4 vorliegt, die den Membranzylinder umgibt. Als Membran wurde eine Cellulosemembran (Cuprophan) verwendet. Bei geeigneten Medien in den Kammern 3, 4 können aber auch z. B. mikroporöse Polypropylenmembranen oder Polyamidmembranen verwendet werden. Das nutzbare Volumen der inneren Kammer 3 betrug 1,6 1, das der äußeren Kammer 4 dagegen 8 1. Die Organismen wurden in die innere Kammer 3, also in den Innenraum des Membranzylinders gebracht. Die äußere Kammer 4 diente der Aufnahme der Dialyseflüssigkeit als weiterer Nährlösung zum Abtransport von das Wachstum hemmenden Stoffen, die über die Membran 2 aus der inneren Kammer 3 in die äußere Kammer 4 gelangten. Gezüchtet wurde ein Stamm von E. coli. Anders als bei den bekannten Verfahren wird die Nährlösung in zwei Komponenten geteilt:

Ein Teil besteht aus Wasser, in dem die notwendigen, wenig Kosten verursachenden Nährsalze gelöst sind. Diese Salzlösung wird in das Vorratsgefäß 6 gefüllt.

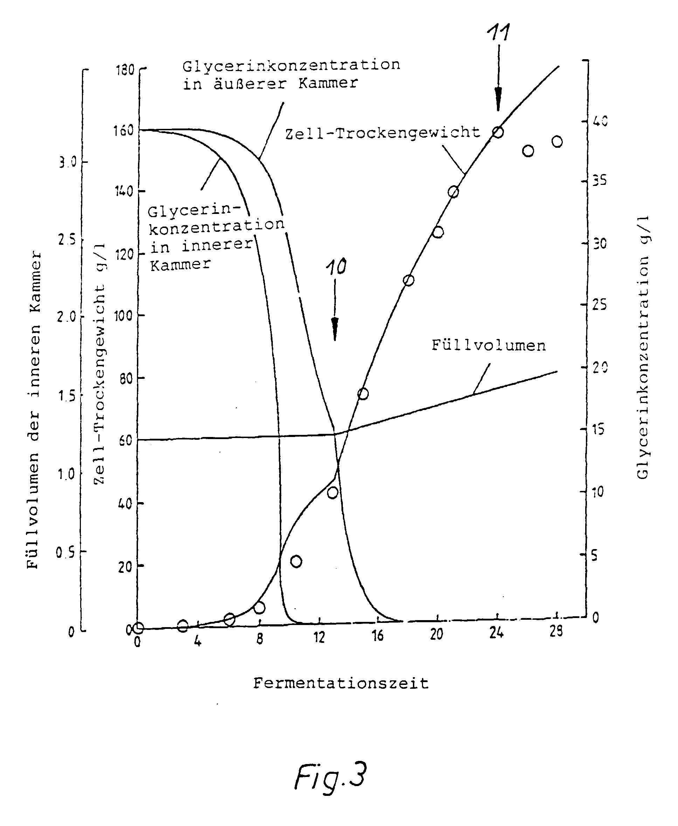

Der zweite Teil wird durch die Kohlenstoffquelle dargestellt, die die wesentlichen Rohstoffkosten verursacht. Diese wird in das Vorratsgefäß 7 gefüllt. Um dichte Kulturen zu erzeugen, ist es notwendig, die Kohlenstoffquelle möglichst hoch zu konzentrieren. Im konkreten Fall besteht die Kohlenstoffquelle aus unverdünntem Glycerin. Falls die Organismen mit weiteren teuren Nährkomponenten, wie z. B. Vitaminen oder anderen wachstumsfördernden Stoffen versorgt werden müssen, können diese in einem weiteren Vorratsgefäß 8 untergebracht werden. Gestartet wurde die Fermentation konventionell, d. h. beide Räume erhielten eine Nährlösung bestehend aus etwa 40 g/l Glycerin und den notwendigen Nährsalzen. Nach etwa 12 h war das Glycerin in der inneren Kammer verbraucht (vergl. Diagramm Fig. 3) und wurde durch das noch in der äußeren Kammer 4 verbliebene Glycerin über die Membran 2 nachgeliefert. Nach 13 h Fermentationszeit wurde nun sowohl die Dialyse durch Pumpen von Salzlösung aus dem Vorratsgeäß 6 in die äußere Kammer 4 und Abpumpen von Hemmstoffen enthaltender Salzlösung in das Gefäß 9 als auch die direkte Fütterung mit Glycerin aus dem Vorratsgefäß 7 in die innere Kammer 3 angeschaltet. Das tatsächliche Wachstum folgt praktisch vollkommen dem, was durch theoretische Berechnung vorermittelt wurde. Das Ergebnis der theoretischen Berechnung ist in Fig. 3 durch die ausgezogenen Kurven dargestellt, während die offenen Kreise Meßwerte des Versuchs zeigen.

Da die theoretische Berechnung Produkthemmung nicht beinhaltet, ist gezeigt, daß mit dem vorgestellten Verfahren die Produkthemmung praktisch vollständig ausgeschaltet werden kann. Die erreichten Konzentrationen (Fig. 3, Zeitpunkt 11) von E. coli lagen bei 160 g/l, gemessen in getrocknetem Zustand.

Da zum Zeitpunkt der direkten Fütterung mit Glycerin (vergl. Fig. 3, Zeitpunkt 10) bereits eine hohe Mikroorganismendichte besteht, wurde das Glycerin unmittelbar aufgenommen. Die Konzentration von Glycerin in der inneren Kammer 2 war nahe Null, deswegen wurde kein Glycerin in die äußere Kammer 4 abgegeben. Da außerdem zu diesem Zeitpunkt auch die Glycerinkonzentration in der äußeren Kammer stark gesunken ist (vergl. Fig. 3), wurde auch durch die Dialyse mit Salzlösung nur wenig Glycerin verloren. Auf diese Weise ergab sich, bezogen auf die produzierte Zellmasse, eine günstige Bilanz bezüglich des verbrauchten Glycerins.

### Versuch 2:

Die Räume für die Organismen und die Dialyseflüssigkeit waren räumlich getrennt und über ein Membranmodul miteinander gekoppelt. In einem mit dieser Anordnung durchgeführten Experiment, analog zu dem oben beschriebenen Versuch 1, wurden Zelldichten erzielt, die bei etwa 100 g/l lagen. Offenbar hatte die mangelhafte Versorgung der außerhalb des Fermenters im Kreislauf befindlichen Zellen einen negativen Einfluß.

### Versuch 3:

In einem weiteren Experiment entsprechend des Versuches 1 wurde auf den Austausch der in der äußeren Kammer 4 enthaltenen Nährlösung verzichtet. Statt dessen wurden in die äußere Kammer 4 des Dialyse-Bioreaktors 1 100 g eines bestimmten Harzes (XAD-Adsorber, Typ. 8, Partikelgröße 0,3 bis 1 mm) eingegeben. Wie das Ergebnis zeigte, wurden wachstumshemmende Stoffwechselprodukte bis zu einem bestimmten Maße von dem Harz gebunden und unschädlich gemacht. Erzielt wurden Organismendichten von etwa 170 g/l, ohne daß Nährlösung verloren ging.

Da im Versuch 3 die in der äußeren Kammer befindliche Salzlösung nicht ausgetauscht wird, kann es notwendig sein, die Mikroorganismen zusätzlich mit konzentrierter Salzlösung zu versorgen.

Die in den Versuchen 1 bis 3 angegebenen Verfahren beschreiben einen absatzweise betriebenen Prozeß (batch-Verfahren). Die in der inneren Kammer 3 befindlichen Mikroorganismen werden bis zu einer gewissen Dichte angezogen und dann abgeerntet. Anschließend wird der Prozeß, wie beschrieben, neu in Gang gesetzt. Diese Verfahrensweise wird bevorzugt, wenn die Bildung eines erwünschten Produktes in einer bestimmten Phase des Prozesses induziert wird. Nach Produktion der erwünschten Substanz wird diese mit den Zellen abgeerntet.

Ist das Prozeßziel jedoch der Mikroorganismus selbst oder ein Produkt, das fortlaufend produziert wird, dann ist auch ein kontinuierlicher Betrieb möglich. In diesem Fall ist der Beginn des Verfahrens identisch mit dem des absatzweise betriebenen Prozesses. Bei einer bestimmten Organismendichte wird dann kontinuierlich über eine in Fig. 1 nicht näher dargestellte Ernteleitung 21 Organismen enthaltende Flüssigkeit entnommen.

Damit das Füllvolumen der inneren Kammer 3 konstant bleibt, wird gleichzeitig, ebenfalls kontinuierlich, aus dem Vorratsgefäß 7 Nährmedium nachgefüllt. Die Konzentration der Kohlenstoffquelle im Vorratsgefäß 7 ist im Vergleich zu der beim batch-Prozeß eingesetzten Konzentration niedriger. Gibt man im kontinuierlichen Prozeß die erwünschte Zell- bzw. Produktkonzentration vor, so kann die im kontinuierlichen Fall zuzuführende Nährstoffkonzentration aus den entsprechenden Verbrauchsdaten der Zellen (Koeffizienten für Substratausbeute und Erhaltungsstoffwechsel) vorausberechnet werden.

Die Pumprate, mit der die Nährlösung zugepumpt wird, muß sich nach dem aktuellen Nährstoffbedarf der zu kultivierenden Organismen richten. Dies gilt gleichermaßen für den kontinuierlichen und den diskontinuierlichen Fall. Bei aerob, d. h. unter Sauerstoffverbrauch wachsenden Organismen, dürfen Nährstoffe beispielsweise maximal nur in der Menge zugefüttert werden, wie es dem technisch zu bewerkstelligenden Sauerstoffeintrag entspricht. Überschreitet man im Fall hoher Zellkonzentrationen die zulässige Zufütterrate, so unterschreitet die im Kulturmedium befindliche Sauerstoffkonzentration einen bestimmten kritischen Wert mit der Folge einer unerwünschten Stoffwechseländerung. Die kritische Zupumpgeschwindigkeit kann im gegebenen Fall experimentell, etwa durch Messung der Sauerstoffkonzentration in der inneren Kammer 3 bestimmt werden.

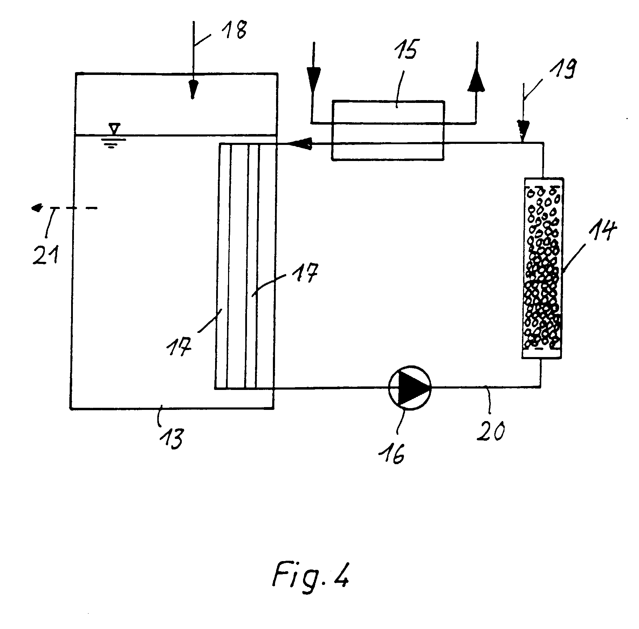

Soll in einem technischen Verfahren die Fähigkeit des Harzmaterials, wachstumshemmende Stoffe zu binden, genutzt werden, so wäre z. B. eine Anordnung nach Fig. 4 denkbar. In einem Mikroorganismen enthaltenden Reaktor 13 werden zylinderförmige, parallel geschaltete Membranmodule 17 angebracht, die kontinuierlich über eine Pumpe 16 von Salzlösung durchströmt werden. Eine Regeneration der Salzlösung, d. h. die Entfernung wachstumshemmender Stoffe, findet in einer Harz enthaltenden Säule 14 statt. Gleichzeitig kann in diesen Kreislauf 20 ein Wärmetauscher 15 geschaltet werden, der für die Abfuhr eines Teiles der entstandenen Wärme sorgt. Wärme entsteht durch die im Reaktor ablaufende exotherme Reaktion und durch Energie, die durch das Rührwerk eingebracht wird. Durch den Zulauf 18 ist angedeutet, daß die Kultur mit einer hoch konzentrierten Kohlenstoffquelle und zusätzlich bei Bedarf mit einer konzentrierten Lösung bestimmter Wuchsstoffe, wie Vitaminen, versorgt wird. Verbrauchte Nährsalze können in den Dialysekreislauf durch eine Zuleitung 19 eingespeist werden. Eine weitere Entnahmeleitung 21 ermöglicht einen wie oben beschriebenen kontinuierlichen Betrieb.

Eine weitere Verfahrensvariante würde darin bestehen, auf die Membranen 17 sowie den außenliegenden Flüssigkeitskreislauf 20 insgesamt zu verzichten und das Tauscherharz direkt in den Reaktor zu bringen und dort zu suspendieren. Nach der Fermentation kann das Harz mittels einer Trennvorrichtung von den produzierten Organismen getrennt werden.

Wie eingangs dargelegt können die hier beispielhaft beschriebenen Verfahrensvarianten bei Kulturen zum Einsatz kommen, die aus Mikroorganismen oder aus pflanzlichen Zellen oder aus tierischen Zellen bestehen.

## Patentansprüche

1. Verfahren zur Erzeugung hoher Zelldichten in einem Reaktionssystem, das aus mindestens zwei Kammern besteht, die über mindestens eine Membran miteinander gekoppelt sind und bei dem die mit Nährstoffen zu versorgende Kultur von Mikroorganismen oder pflanzlichen oder tierischen Zellen sich in der einen Kammer befindet und die wachstumshemmenden Stoffwechselprodukte der Kammer aus dieser Kammer durch die Membran in die Lösung der anderen Kammer gelangen, gekennzeichnet, durch die Kombination folgender Merkmale:
a) die mindestens eine Membran wird direkt in die die Kultur von Mikroorganismen, pflanzlichen Zellen oder tierischen Zellen enthaltende Kammer integriert
b) die mindestens eine andere Kammer enthält nach der Anfahrphase des Reaktionssystems eine den osmotischen Ausgleich über die mindestens eine Membran ermöglichende salzhaltige Lösung ohne Kohlenstoffquelle
c) in die die Kultur von Mikroorganismen, pflanzlichen Zellen oder tierischen Zellen enthaltende Kammer wird zur Erzeugung schnellen Wachstums der Kultur in technisch zu bewerkstelligendem Umfang eine Menge Sauerstoff eingetragen
d) in die die Kultur von Mikroorganismen, pflanzliche Zellen oder tierische Zellen enthaltende Kammer wird eine eine kohlenstoffhaltige Substanz enthaltende flüssige hochkonzentrierte Nährlösung in einer Menge zugeführt, die vom Sauerstoffeintrag abhängig ist.

2. Verfahren nach Anspruch 1, dadurch gekennzeichnet, daß zunächst in der Anfahrphase jeder Kammer eine die Nährstoffe enthaltende Nährlösung zugeführt wird, und daß dann, wenn die Nährstoffversorgung der Kultur durch die mindestens eine Membran nicht mehr ausreicht, der die Kultur enthaltenden Kammer die hochkonzentrierte Nährlösung direkt zugeführt wird.

3. Verfahren nach Anspruch 1 oder 2, dadurch gekennzeichnet, daß aus der die Kultur von Mikroorganismen, pflanzlichen Zellen oder tierischen Zellen enthaltenden Kammer in die salzhaltige Lösung eingedrungene wachstumshemmende Stoffwechselprodukte mit der salzhaltigen Lösung aus der die salzhaltige Lösung aufnehmenden Kammer entfernt werden.

4. Verfahren nach Anspruch 1 oder 2, dadurch gekennzeichnet, daß die in die salzhaltige Lösung eingedrungenen wachstumshemmenden Stoffwechselprodukte durch Zugabe eines Adsorberharzes in die salzhaltige Lösung an das Adsorberharz gebunden und durch dieses neutralisiert werden.

5. Verfahren nach Anspruch 4, dadurch gekennzeichnet, daß als Adsorberharz ein XAD-Adsorber verwendet wird.

6. Verfahren nach Anspruch 1, dadurch gekennzeichnet, daß die hochkonzentrierte Nährlösung zusätzlich Vitamine oder andere das Wachstum fördernde Substanzen aufhält.

7. Verfahren nach Anspruch 1, dadurch gekennzeichnet, daß als hochkonzentrierte Nährlösung Glycerin verwendet wird.

8. Verfahren nach Anspruch 1, dadurch gekennzeichnet, daß als salzhaltige Lösung Wasser mit eingemischten Nährsalzen verwendet wird.

9. Verfahren nach Anspruch 1, dadurch gekennzeichnet, daß den die salzhaltige Lösung enthaltenden Kammern zusätzliche Nährsalze zugeführt werden, die durch das Wachstum der Kultur verbraucht werden.

10. Verfahren nach Anspruch 1, 2 bis 9, dadurch gekennzeichnet, daß die Kultur in eine durch eine zylinderförmige Membran gebildete innere Kammer eingebracht wird, die in der mit der weiteren salzhaltigen Lösung gefüllten äußeren Kammer des Reaktors ausgebildet ist.

11. Verfahren nach Anspruch 1, bei dem die salzhaltige Lösung mit eingedrungenen wachstumshemmenden Stoffwechselprodukten aus dem Reaktionssystem abgeführt wird, dadurch gekennzeichnet, daß die salzhaltige Lösung wieder in das Reaktionssystem zurückgepumpt wird, wobei in den Flüssigkeitskreislauf eine ein Adsorberharz enthaltende Säule geschaltet ist.

12. Verfahren nach Anspruch 11, dadurch gekennzeichnet, daß in den Flüssigkeitskreislauf zusätzlich ein Wärmetauscher geschaltet ist.

13. Verfahren nach Anspruch 1, dadurch gekennzeichnet, daß die die salzhaltige Lösung enthaltender Kammem als parallel geschaltete und miteinander verbundene Membranmodule ausgebildet sind.

14. Verfahren nach einem der Ansprüche 1 bis 4, dadurch gekennzeichnet, daß in sich wiederholender Weise die zu versorgende Kultur bis zu einer bestimmten Dichte angezogen und dann abgeerntet wird.

15. Verfahren nach einem der Ansprüche 1 bis 4, dadurch gekennzeichnet, daß die zu versorgende Kultur bis zu einer bestimmten Dichte angezogen und dann kontinuierlich aus der die Kultur enthaltenden Kammer Organismen enthaltende Flüssigkeit entnommen und in diese Kammer kontinuierlich die gleiche Menge flüssiges Nährmedium eingebracht wird.

16. Vorrichtung zur Durchführung des Verfahrens nach den Ansprüchen 1 bis 15 mit einem Reaktor (13), in dessen Inneren ein Membranmodul (17) angeordnet ist, durch das eine salzhaltige Dialyseflüssigkeit geführt wird, die die wachstumshemmenden Stoffwechselprodukte aufnimmt und abführt, dadurch gekennzeichnet, daß im Flüssigkeitskreislauf (20) des Membranmoduls (17) eine Adsorberharz enthaltende Säule (14) angeordnet ist.

17. Vorrichtung nach Anspruch 16, dadurch gekennzeichnet, daß mindestens zwei parallel geschaltete Membranmodule (17) in dem Reaktor (13) angeordnet sind.

18. Vorrichtung nach Anspruch 16 und 17, dadurch gekennzeichnet, daß in dem Flüssigkeitskreislauf (20) ein Wärmetauscher angeordnet ist.

19. Vorrichtung nach Anspruch 16 bis 18, dadurch gekennzeichnet, daß in dem Reaktor (13) ein Rührwerk angeordnet ist.

## Claims

1. A method of producing high cell densities in a reaction system consisting of at least two chambers coupled with one another by means of a membrane and in which the culture of micro-organisms or plant or animal cells to be fed with nutrients are placed in one chamber and the growth-inhibiting reaction products in the chamber are able to pass out from this chamber through the membrane and into the solution in the other chamber, characterised by the following features in combination:
a) a membrane, of which there is at least one, is directly integrated in the chamber containing the culture of micro-organisms, plant cells or animal cells
b) the other chamber, of which there is at least one, contains a salt solution containing no carbon source which, after the start-up phase of the reaction system, will enable the osmotic balance to be established through the at least one membrane
c) a quantity of oxygen is introduced into the chamber containing the culture of micro-organisms, plant cells or vegetable cells in order to produce rapid growth of the culture on a technically viable scale
d) a highly concentrated liquid nutrient containing a carbon substance is fed into the chamber containing the culture of micro-organisms, plant cells or animal cells in a quantity that is dependent on the oxygen input.

2. A method as claimed in claim 1, characterised in that a nutrient solution containing the nutrients is initially fed into each chamber in the start-up phase and, when the supply of nutrient is no longer sufficient to feed the culture through the at least one membrane, the highly-concentrated nutrient solution is fed directly into the chamber containing the culture.

3. A method as claimed in claim 1 or 2, characterised in that growth-inhibiting reaction products that have penetrated the salt solution from the chamber containing the culture of micro-organisms, plant cells or animal cells are removed, along with the salt solution, from the chamber housing the salt solution.

4. A method as claimed in claim 1 or 2, characterised in that an adsorbing resin is added to the salt solution so that the growth-inhibiting reaction products that have penetrated the salt solution bond with the adsorbing resin and are neutralised thereby.

5. A method as claimed in claim 4, characterised in that an XAD-adsorber is used as the adsorbing resin.

6. A method as claimed in claim 1, characterised in that the highly concentrated nutrient solution additionally contains vitamins or other growth-promoting substances.

7. A method as claimed in claim 1, characterised in that glycerine is used as the highly concentrated nutrient solution.

8. A method as claimed in claim 1, characterised in that water mixed with nutrient salts is used as the salt solution.

9. A method as claimed in claim 1, characterised in that additional nutrient salts are fed into the chambers containing the salt solution, which will be consumed by the growth of the culture.

10. A method as claimed in claim 1, 2 to 9, characterised in that the culture is placed in an inner chamber, formed by means of a cylindrically shaped membrane, and designed such that it is inside the outer chamber of the reactor filled with the additional salt solution.

11. A method as claimed in claim 1, in which the salt solution is fed, together with the growth-inhibiting reaction products, out of the reaction system, characterised in that the salt solution is pumped back into the reaction system, wherein a column containing an adsorbing resin is connected into the fluid circuit.

12. A method as claimed in claim 11, characterised in that a heat exchanger is also connected into the fluid circuit.

13. A method as claimed in claim 1, characterised in that the chambers containing the salt solution are designed as membrane modules connected in parallel and joined to one another.

14. A method as claimed in one of claims 1 to 4, characterised in that the culture to be fed is grown to a given density and then drawn off in batches.

15. A method as claimed in one of claims 1 to 4, characterised in that the culture to be fed is grown to a given density and the fluid containing the organisms is then continuously removed from the chamber containing the culture and the same quantity of liquid nutrient medium is fed continuously into this chamber.

16. A device for implementing the method as claimed in claims 1 to 15 having a reactor (13), in the interior of which a membrane module (17) is arranged, through which a dialysis liquid containing salt is fed, which picks up and feeds away the growth-inhibiting reaction products, characterised in that a column (14) containing an adsorbing resin is incorporated in the fluid circuit (20) of the membrane module (17).

17. A device as claimed in claim 16, characterised in that at least two membrane modules (17) are provided in the reactor (13) and connected in parallel.

18. A device as claimed in claim 16 and 17, characterised in that a heat exchanger is provided in the fluid circuit (20).

19. A device as claimed in claim 16 to 18, characterised in that a stirring mechanism is arranged in the reactor (13).

## Revendications

1. Procédé d'obtention de densités élevées de cellules dans un système réactionnel constitué d'au moins deux compartiments qui sont reliés entre eux par l'intermédiaire d'au moins une membrane, dans lequel la culture de microorganismes ou de cellules végétales ou animales qui doit être alimentée en nutriments se trouve dans l'un des compartiments et les produits du métabolisme inhibant la croissance formés dans ce compartiment sont transférés à partir de celui-ci, à travers la membrane, dans la solution de l'autre compartiment, caractérisé par la combinaison des caractéristiques suivantes :
a) la au moins une membrane est intégrée directement dans le compartiment contenant la culture de microorganismes ou de cellules végétales ou animales
b) le au moins un autre compartiment contient, après la phase de démarrage du système réactionnel, une solution saline dépourvue de source de carbone, permettant l'établissement d'un équilibre osmotique par l'intermédiaire de la au moins une membrane,
c) dans le compartiment contenant la culture de microorganismes ou de cellules végétales ou animales on introduit, pour l'obtention d'un avancement rapide de la culture dans des conditions réalisables à l'échelle industrielle, une certaine quantité d'oxygène
d) dans le compartiment contenant la culture de microorganismes ou de cellules végétales ou animales, on introduit une solution nutritive liquide à concentration élevée, contenant une substance carbonée, en une quantité qui est proportionnelle à la quantité d'oxygène fournie.

2. Procédé selon la revendication 1, caractérisé en ce que, dans la phase de démarrage, on introduit d'abord dans chacun des compartiments une solution nutritive contenant les nutriments et que, lorsque l'apport de nutriments fourni à la culture à travers la au moins une membrane n'est plus suffisant, on introduit la solution nutritive à concentration élevée directement dans le compartiment contenant la culture.

3. Procédé selon la revendication 1 ou 2, caractérisé en ce que les produits du métabolisme inhibant la croissance, qui se sont infiltrés dans la solution saline à partir du compartiment contenant la culture de microorganismes ou de cellules végétales ou animales, sont éliminés du compartiment recevant la solution saline en même temps que la solution saline est éliminée.

4. Procédé selon la revendication 1 ou 2, caractérisé en ce que les produits du métabolisme inhibant la croissance qui se sont infiltrés dans la solution saline sont neutralisés par addition à la solution saline d'une résine adsorbante sur laquelle ces produits sont adsorbés.

5. Procédé selon la revendication 4, caractérisé en ce que, en tant que résine adsorbante, on utilise un XAD-Adsorber.

6. Procédé selon la revendication 1, caractérisé en ce que la solution nutritive à concentration élevée contient en outre des vitamines ou d'autres substances favorisant la croissance.

7. Procédé selon la revendication 1, caractérisé en ce que, en tant que solution nutritive à concentration élevée, on utilise de la glycérine.

8. Procédé selon la revendication 1, caractérisé en ce que, en tant que solution saline, on utilise de l'eau dans lequel on a mélangé des sels nutritifs.

9. Procédé selon la revendication 1, caractérisé en ce que dans le compartiment contenant la solution saline, on introduit des sels nutritifs supplémentaires qui sont consommés au cours de l'avancement de la culture.

10. Procédé selon les revendications 1, 2 à 9, caractérisé en ce que la culture est introduite dans un compartiment intérieur constitué d'une membrane cylindrique, formé dans le compartiment extérieur du réacteur, rempli du reste de la solution saline.

11. Procédé selon la revendication 1, dans lequel la solution saline, avec les produits du métabolisme inhibant la croissance qui s'y sont infiltrés, est évacuée du système réactionnel, caractérisé en ce que la solution saline est recyclée par pompage dans le système réactionnel, une colonne contenant une résine adsorbante étant incorporée dans le circuit de recirculation du liquide.

12. Procédé selon la revendication 11, caractérisé en ce qu'un échangeur calorique est inclus en outre dans le circuit de recirculation du liquide.

13. Procédé selon la revendication 1, caractérisé en ce que les compartiments contenant la solution saline sont conçus sous la forme de modules à membrane montés en parallèle et reliés entre eux.

14. Procédé selon l'une quelconque des revendications 1 à 4, caractérisé en ce que de manière répétitive, la culture à alimenter est amenée jusqu'à une densité déterminée et ensuite, elle est amenée à maturation.

15. Procédé selon l'une quelconque des revendications 1 à 4, caractérisé en ce que la culture à alimenter est amenée jusqu'à une densité déterminée et ensuite, elle est évacuée en continu du compartiment contenant la culture et la même quantité de milieu nutritif liquide est introduite en continu dans ce compartiment.

16. Dispositif destiné à la mise en oeuvre du procédé selon les revendications 1 à 15, comportant un réacteur (13) à l'intérieur duquel est disposé un module à membrane (17) à travers lequel on fait circuler un liquide de dialyse salin qui absorbe et évacue les produits du métabolisme inhibant la croissance, caractérisé en ce qu'une colonne contenant une résine adsorbante (14) est incluse dans le circuit de recirculation (20) du liquide du module à membrane (17).

17. Dispositif selon la revendication 16, caractérisé en ce qu'au moins deux modules à membrane (17) montés en parallèle sont disposés dans le réacteur (13).

18. Dispositif selon la revendication 16 ou 17, caractérisé en ce qu'un échangeur calorique est monté dans le circuit (20) de recirculation du liquide.

19. Dispositif selon la revendication 16 à 18, caractérisé en ce remueur est monté dans le réacteur (13).
